# FASCICULE DE BREVET EUROPEEN

(11) **EP 0 472 531 B1**
(45) Date de publication et mention de la délivrance du brevet: **16.08.1995**
(21) Numéro de dépôt: 90905558.4
(22) Date de dépôt: 23.03.1990
(51) Int. Cl.: A61K 35/78

(54) **COMPOSITION A BASE DE PROANTHOCYANIDOLS; LEUR APPLICATION PHARMACOLOGIQUE**
ZUSAMMENSETZUNG AUF DER BASIS VON PROANTHOCYANIDOLEN UND IHRE PHARMAZEUTISCHE ANWENDUNG
PROANTHOCYANIDOL-BASED COMPOSITION AND ITS PHARMACOLOGICAL APPLICATION

(30) Priorité: 12.05.1989 FR 8906282; 09.06.1989 FR 8907654; 11.10.1989 FR 8913297
(43) Date de publication de la demande: 04.03.1992
(73) Titulaire: Cariel, Léon, F-75016 Paris (FR); INSTITUT DES SUBSTANCES VEGETALES, F-63960 Veyre-Monton (FR)
(72) Inventeur: Jean, Daniel, F-63270 Vic-le-Comte (FR)
(74) Mandataire: Orès, Bernard
(86) Numéro de dépôt international: FR9000200
(87) Numéro de publication internationale: WO9013304

(56) Documents cités:
- EP-A- 0 025 649
- EP-A- 0 216 936
- US-A- 4 698 360
- PATENT ABSTRACTS OF JAPAN, Volume 9, No. 59 (C-270)(1782), 15 Mars 1985; & JP-A-59 196 884
- PATENT ABSTRACTS OF JAPAN, Volume 11, No. 242 (C-438)(2689), 7 Aout 1987; P JP-A-62 048 677
- CHEMICAL ABSTRACTS, Volume 100, No. 16, 16 Avril 1984, Columbus, Ohio, US; S.H. MISRA et al.: "Pharmaceutical Studies on Starches of Some Zingiberaceous Rhizomes", voir page 355* Abrege 126823x

## Description

L'invention concerne des compositions à base de proanthocyanidols ainsi que leur application dans des compositions pharmaceutiques.

Les Proanthocyanidols appartiennent à la famille des polyphénols connus pour leurs facultés de se fixer, à des degrés très divers, sur les protéines.

Les polyphénols participent de façon générale à la formation de tanins présents notamment chez certains végétaux.

L'invention se rapporte à des compositions contenant des classes spécifiques de tanins, appelés tanins catéchiques (synonyme de proanthocyanidols ou procyanidols). Elle se rapporte plus précisément à des polymères de tanin catéchique.

D'une façon générale on connaissait jusqu'à présent la capacité de certains extraits bruts de végétaux comprenant divers tanins, de présenter une activité antiseptique, voire quelquefois anti-virale.

Certains tanins présents sous forme d'oligomères, étaient utilisés pour leurs propriétés veinotoniques. Jusqu'à présent, les tanins catéchiques ou autres, pharmaceutiquement utilisables étaient en général constitués par des oligomères (de poids moléculaires généralement inférieurs à 1000). On écartait systématiquement les tanins de poids moléculaires (PM) élevés, notamment les polymères. Par exemple, on rappelle que pour les tanins galliques (autre famille de tanins présents par exemple chez les végétaux) la cytotoxicité augmente avec le poids moléculaire, ce qui ne favorise pas leur utilisation.

Les inventeurs ont maintenant constaté que des tanins catéchiques de poids moléculaire élevé, se présentant sous la forme de polymères, peuvent s'avérer intéressants pour leurs propriétés pharmacologiques.

L'invention concerne à cet égard une composition à base de proanthocyanidols, notamment d'origine végétale, caractérisée par une proportion pondérale d'au moins 50% de molécules ayant des poids moléculaires apparents supérieurs à 2000, cette proportion étant constituée d'au moins 40% de proanthocyanidols, ce pourcentage étant exprimé en fonction tannante, et en ce que ladite composition est essentiellement exempte:
- d'essences aromatiques et notamment d'huiles essentielles, de résines et autres substances lipophiles,
- de sucres polymérisés tels que l'amidon, les gommes, les dérivés cellulosiques,
- de sucres oligomères tels que le saccharose, le glucose, le levulose.

Des méthodes particulièrement adaptées pour la sélection de compositions comprenant des proanthocyanidols et répondant à la définition ci-dessus sont par exemple les suivantes:
a/ Pour caractériser le niveau de la fonction tannante des proanthocyanidols des compositions ci-dessus, on pourra mettre en oeuvre le dosage suivant qui comprend d'une part un dosage de la fonction polyphénolique, d'autre part, un dosage de la fonction tannante.

### 1^{·}/ -Dosage de la fonction polyphénolique:

Dans ce dosage on utilise la propriété des polyphénols de réduire l'acide phosphotungstique. Le dosage est réalisé selon les normes de la pharmacopée française 9^{·} édition.

### Réactif phosphotungstique (A):

On chauffe à reflux 10g de tungstate de sodium avec 8ml d'acide orthophosphorique et 75ml d'eau désionnisée pendant 3 heures. On laisse refroidir et on complète à 100ml avec de l'eau désionnisée. Solution de carbonate de sodium à 15% dans de l'eau désionnisée: réactif B.

On pèse 50mg de l'échantillon contenant le proanthocyanidol à doser et on les dissout dans 10ml d'eau mesuré exactement.

On prélève 2ml de cette solution et on la dilue à 25ml dans une fiole jaugée, (solution C).
Dans une fiole jaugée de 50ml, on place:
Solution C: 5 ml
Réactif A: 1ml
Réactif B: q.s.50ml.

On lit la densité optique (D.O.) à 715nm, 2 minutes exactement après le mélange du dernier réactif. On opère de même avec une gamme de quantités croissantes de pyrogallol (0 à 50mg) comme références et l'on mesure la densité optique obtenue avec chacune d'entre elles. La teneur en proanthocyanidol de l'échantillon étudié, est exprimé par référence à la quantité de pyrogallol (quantité mesurée) qui conduirait, dans les mêmes conditions d'analyse, à la même mesure de D.O. La détermination de cette quantité de pyrogallol se fait par référence aux résultats des mesures faites dans les conditions sus-indiquées avec les quantités croissantes de pyrogallol utilisées à titre de références.

### 2^{·}/ -Dosage de la fonction tannante:

Une solution aqueuse de proanthocyanidol préparée comme la solution C ci-dessus. On effectue alors le dosage de la fonction polyphénolique comme indiqué ci-dessus. On agite ensuite 5ml de la solution pendant une heure à température ambiante avec 1g de poudre de peau préchromée (Merck réf. 4332); puis la solution est filtrée sur papier.

On opère ensuite un dosage de la fonction polyphénolique totale sur le filtrat comme ci-dessus. La fonction tannante représente la différence entre la valeur trouvée pour les polyphénols totaux et la valeur trouvée sur le filtrat, séparé par la poudre de peau.
b/ Pour déterminer la fraction des proanthocyanidols dont le poids moléculaire apparent est supérieur à 2000, on pourra utiliser la méthode de détermination du poids moléculaire suivante:
   On opère dans un premier temps une chromatographie haute performance d'exclusion sur gel »Bondagel 125 (Waters) dans un solvant aprotique, contre des témoins de polystyrène (Aldrich)
Débit: 0,5 ml/minute en isocratique.
Solvant: Chloroforme-diméthylsulfoxyde 50-50 (v/v)
Détection: D.O. à 283nm
Concentration des témoins: 1mg/ml dans le même solvant Concentration du proanthocyanidol: 0,05mg/ml dans le même solvant
Poids moléculaires des témoins: 184.200, 50.000, 24.150, 7.025, 4.136 et 2.000.

Le témoin de 184.200 de poids moléculaire est utilisé comme témoin d'exclusion total sur le gel. En effet ce type de gel ne retient pas les poids moléculaires supérieurs à 50.000. On calcule ensuite le temps de rétention relatif de chaque échantillon par rapport au témoin totalement exclu.

On trace la courbe de correspondance entre les logarithmes décimaux des poids moléculaires des témoins et les temps relatifs de rétention correspondants. Le poids moléculaire des proanthocyanidols présents est apprécié par comparaison du temps de rétention mesuré pour eux et des temps de rétention des témoins. Ces méthodes sont données uniquement à titre d'exemple.

D'autres méthodes permettant de déterminer la proportion de proanthocyanidols dans une composition donnée et conduisant également à donner la répartition en poids des proanthocyanidols présents, sont naturellement adaptées à la réalisation de l'invention.

L'invention vise également une composition à base de proanthocyanidols répondant aux définitions ci-dessus, caractérisée en ce qu'elle est en outre essentiellement exempte:
- de protéines végétales,
- d'acide gallique et de tanins galliques,
- de flavonoïdes n'appartenant pas à la famille des flavanes-diols,
- de flavanes-diols oligomères,
- d'acides phénols.

Selon un premier mode de réalisation avantageux de l'invention, les compositions de l'invention sont caractérisées en ce qu'elles sont constituées d'au moins environ 95% en poids sec de proanthocyanidols.

Selon un autre aspect avantageux de l'invention, ces compositions à base de proanthocyanidols sont caractérisées en ce que les proanthocyanidols ont un poids moléculaire compris entre environ 2000 ± 500 et environ 55000 ± 5000.

Une composition particulièrement intéressante comprend de préférence des proanthocyanodols de poids moléculaire d'environ 45000 ± 5000.

D'autres compositions particulièrement intéressantes sont des compositions comprenant des proanthocyanidols ayant un poids moléculaire d'environ 5000 ± 500, ou des compositions comprenant des proanthocyanidols ayant un poids moléculaire d'environ 3000 ± 300.

La détermination de la pureté du proanthocyanidol polymère, dans un intervalle de poids moléculaire choisi, peut être effectuée en utilisant une chromatographie sur gel, à l'aide d'une membrane organique ayant un seuil de coupure déterminé, par exemple une membrane de polyéthersulfone ou de difluorure de polyvinylidène. Des détails concernant le protocole mis en oeuvre sont donnés dans les exemples.

Les compositions adaptées à la réalisation de l'invention sont selon une autre définition, caractérisées en ce qu'elles comprennent des proanthocyanidols sous forme de polymères, répondant à la formule suivante: 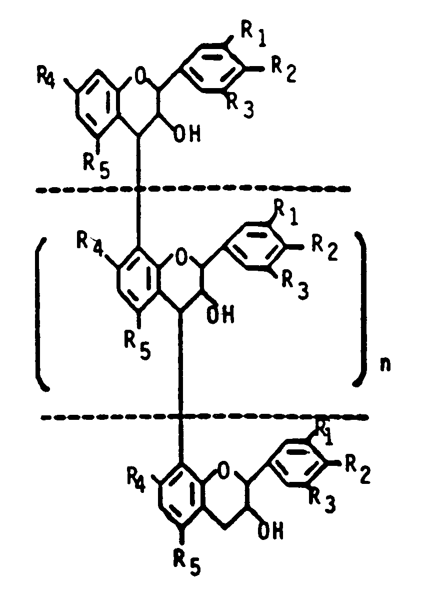
dans laquelle n est un entier supérieur ou égal à 3 et de préférence compris entre 3 et 350,
R₁, R₂, R₃, R₄ et R₅ sont ensemble ou séparément, l'hydrogène, un groupement hydroxyle ou un groupement méthoxy, et de préférence en ce qu'il s'agit de polymères de procyanidols dans lequel R₁, R₂, R₄ et R₅ sont des groupements hydroxyle et R₃ est de l'hydrogène, ou de polymères de prodelphinidols dans lequel R₁, R₂, R₃, R₄ et R₅ sont des groupes hydroxyle ou en ce qu'il s'agit de polymères de promalvidols dans lequel R₁ et R₃ sont des groupes méthoxy et R₂, R₄ et R₅ sont des groupements hydroxyle.

Les polymères de proanthocyanidols de l'invention peuvent être soit sous forme linéaire, ou encore organisés en réseaux. Les compositions de l'invention peuvent être formées d'un ou de plusieurs types de proanthocyanidols notamment lorsque ces compositions sont des extraits obtenus à partir d'une matière végétale, plusieurs types de polymères peuvent être présents dans la composition et leur répartition n'est pas nécessairement constante selon la matière végétale initiale utilisée.

D'une façon particulièrement intéressante, les compositions de l'invention comprennent des proanthocyanidols présentant une certaine solubilité dans l'eau, fonction du poids moléculaire et la nature de la molécule.

Lorsque ces compositions sont des extraits végétaux, elles peuvent être obtenues à partir de tout matériel végétal de plantes riches en proanthocyanidol de poids moléculaire supérieur à 2000 et susceptibles, au terme de la mise en oeuvre des procédés de dosage des fonctions polyphénoliques et tannantes, de donner des compositions ayant les caractéristiques ci-dessus définies.

Ainsi, à titre d'exemple d'espèces végétales utilisables comme source de proanthocyanidols, on cite les conifères tels que Cupressus sempervirens, Pinus maritima, les zingibéracées telles que Alpinia Galanga, les plantes à procyanidols, à prodelphinidols, telles que Vitis vinifera, Vaccinum myrtillus, Fragaria vesca et des plantes à proanthocyanidols plus rares telles que les plantes à profisetinidols du genre Schinopsis et à proguibourtinidols extraits de divers Acacia.

On peut également citer des plantes de la famille des mimosacées.

L'invention vise naturellement, dans un mode de réalisation préféré, des compositions comprenant plusieurs types d'extraits végétaux satisfaisant aux définitions données.

L'invention concerne également un procédé pour la préparation d'une composition de proanthocyanidols, à partir d'une partie de plante contenant des proanthocyanidols de poids moléculaire supérieur à 2000, ce procédé étant caractérisé en ce qu'il comprend les étapes suivantes:
- décoction ou macération dans un alcool, notamment le méthanol ou l'éthanol, pour éliminer les polysaccharides notamment l'amidon,
- récupération des extraits hydroalcooliques et évaporation à sec, sous pression réduite, suivie éventuellement de lavage et récupération du résidu sec,
- reprise du résidu sec et filtration pour éliminer une partie de la fraction aromatique de l'extrait,
- extraction à contre-courant, à l'aide successivement d'au moins 3 solvants de polarité croissante, notamment l'éther éthylique, l'acétate d'éthyle, le butanol ou le méthanol notamment pour éliminer les essences aromatiques restantes, la plus grande partie possible des oligomères de PM inférieur à 2000 et des monomères de proanthocyanidols et pour récupérer dans la phase alcoolique un extrait végétal contenant les proanthocyanidols, de poids moléculaire supérieur à 2000,
- le cas échéant, la purification de l'extrait obtenu pour éliminer les solvants restants.

L'étape de purification peut consister en une dyafiltration avec de l'eau désionnisée afin d'éliminer la plus grande partie du solvant. Cette opération est par exemple réalisée en réfrigérant le liquide en continu à basse température en particulier à 4°C.

Pour la conservation du produit, une fois le solvant éliminé une possibilité offerte consiste à concentrer le plus possible la solution-suspension aqueuse de proanthocyanidols polymères puis à la lyophiliser. La conservation peut également être réalisée sous forme d'un produit cristallisé à partir de la solution-suspension aqueuse.

L'extraction est de préférence menée de manière à éviter la dégradation des proanthocyanidols. Pour ce faire, il est préférable d'éviter de trop hautes températures et de réduire la quantité d'oxygène en contact avec les composés proanthocyanidoliques. Ainsi, l'extraction et les étapes ultérieures peuvent être réalisées sous atmosphère inerte, par exemple sous azote.

Pour éliminer les proanthocyanidols de poids moléculaire inférieur à 2000, on pourra mettre en oeuvre la méthode suivante: la fraction extraite au moyen des divers solvants utilisés est ultrafiltrée sur une membrane organique et notamment de type membrane de polyéther sulfone, ou une membrane de difluorure de polyvinylidène, dont le seuil de coupure est choisi en fonction de la fraction de proanthocyanidol que l'on souhaite éliminer. En fin d'opération, on obtient une solution concentrée de polymères retenus sur la membrane, et une solution diluée de produits de poids moléculaire inférieur à celui du seuil de coupure.

Ces étapes peuvent être suivies éventuellement d'une étape de lavage jusqu'à obtention d'une teneur inférieure à 10%, avantageusement inférieure à 5%, et de préférence à 1% de produits légers, par rapport à la substance finale obtenue sèche.

Un autre procédé particulièrement avantageux pour la préparation des compositions de proanthocyanidols, à partir d'une partie de plante contenant des proanthocyanidols de poids moléculaire supérieur à 2000, comprend les étapes suivantes:
- décoction ou macération dans un alcool, notamment le méthanol ou l'éthanol, pour éliminer les polysaccharides, notamment l'amidon,
- ultrafiltration des solutions hydroalcooliques sur une membrane organique d'une porosité telle que son seuil de coupure serait de 20000 Daltons pour une protéine globulaire ultrafiltrée dans une solution aqueuse.
- récupération des extraits hydroalcooliques et évaporation à sec, sous pression réduite, suivie éventuellement de lavage et récupération du résidu sec.

Selon un mode de réalisation avantageux de l'invention, le procédé décrit ci-dessus peut être mis en oeuvre à partir d'une matière végétale initiale constituée par des cônes de Cupressus sempervirens, ou à partir de rhizomes frais d'Alpinia galanga.

Les compositions de l'invention sont particulièrement intéressantes dans la mesure où elles sont dépourvues de cytotoxicité et partant peuvent être utilisées dans des compositions pharmaceutiques. De plus, les inventeurs ont constaté la capacité des proanthocyanidols de ces compositions de se fixer sur les récepteurs cellulaires des virus. A cet égard, l'invention concerne des compositions pharmaceutiques comprenant une quantité efficace à titre de principes d'actifs, d'au moins une composition de proanthocyanidols telle que définie précédemment, en association avec un véhicule pharmaceutique acceptable.

De telles compositions peuvent être utilisées selon différents modes d'administration.

A titre d'exemple on peut citer comme voies d'administration adaptées, la voie topique (dermique ou oculaire), la voie rectale, la voie orale.

De façon particulièrement avantageuse, elles seront appliquées sous forme topique, dans ce cas elles comprennent des excipients ou des adjuvants habituellement utilisés pour ce mode d'administration.

Ces compositions pharmaceutiques comprennent avantageusement entre environ 0,1% et environ 5% de principes actifs.

L'utilisation des proanthocyanidols de l'invention dans des compositions pharmaceutiques est intéressante pour les propriétés antivirales de ces molécules. De telles compositions sont particulièrement adaptées pour combattre des virus tels que celui de l'herpès type I, de l'herpès type II, de la varicelle-zona, de l'Influenza A, le virus Echo 25 et le virus HIV, le rotavirus, le cytomégalovirus et le virus de l'hépatite A.

Un autre intérêt des proanthocyanidols de l'invention dans des compositions pharmaceutiques, est qu'ils peuvent être utilisés en tant qu'antiprotéases, notamment en tant qu'antiélastases et anticollagènases. Les inventeurs ont en effet montré qu'ils possèdent la propriété de protéger in vivo le tissu conjonctif contre les enzymes qui le dégrade de façon naturelle. Par conséquent, ils peuvent être utilisés comme agents antiemphysème, veinotonique et vasculoprotecteurs, comme agents de la lutte contre les effets du vieillissement du tissu conjonctif tel que celui du système cardiovasculaire, de la peau, des muqueuses , du pancréas, des gencives, etc...

Les proanthocyanidols de l'invention ont aussi la propriété de renforcer l'attachement de la matrice intercellulaire aux fibroblastes, de stimuler la fibrillogénèse du collagène et de stimuler la synthèse des protéines du parenchyme hépatique.

Un avantage des compositions de l'invention réside dans le fait qu'elles entraînent peu d'effets secondaires néfastes, et sont d'une toxicité très faible.

Une posologie adaptée à l'administration orale consiste à administrer de 0,01/kg/24heures de composition à 0,2/kg/24heures de composition et de préférence environ 0,1g/kg/24 heures.

L'invention vise également des compositions pharmaceutiques comprenant à titre de principe actif, des proanthocyanidols en association avec une ou plusieurs substance(s) choisie(s) parmi les antibiotiques, les antiseptiques, les antiinflammatoires et les antipyrétiques, dès lors que ces substances sont chimiquement compatibles avec les proanthocyanidols.

L'invention vise en outre l'utilisation des compositions de proanthocyanidols ci-dessus pour la fabrication d'un médicament à activité antivirale.

D'autres caractéristiques et avantages de l'invention apparaîtront dans les exemples donnés à titre non limitatif dans la suite. Dans ces exemples, le terme "procyanidol" est également utilisé pour désigner les proanthocyanidols.

### Exemple 1

### Extrait hydrométhanolique de cônes de Cupressus sempervirens

200g de cônes secs concassés de Cupressus sempervirens sont décoctés trois fois de suite pendant une demi-heure par 2000ml de méthanol à 80%. Les extraits hydrométhanoliques sont réunis et évaporés à sec sous pression réduite. On reprend ce résidu par 200ml d'eau distillée à 20°C environ. On filtre. On reprend l'insoluble par 200ml d'eau distillée à 20°C environ. On filtre. Les filtrats sont réunis. Le résidu de cette solution est d'environ 33g soit un rendement de 16,5%.

### 1.2 - Fraction procyanidolique

La solution aqueuse, obtenue précédemment est ajustée à 400ml par de l'eau distillée et extraite à contre-courant par 5 fois 100ml d'éther éthylique. La phase éthérée est éliminée, l'éther éthylique est chassé de la phase aqueuse par évaporation sous pression réduite. On complète le volume de la solution aqueuse à 400ml par de l'eau distillée, puis on extrait à contre-courant par 5 fois 100ml d'acétate d'éthyle.

La phase organique est éliminée. On chasse l'acétate d'éthyle de la solution aqueuse par évaporation sous pression réduite. On complète le volume de la solution à 400ml par de l'eau distillée, puis on l'extrait à contre-courant par 5 fois 100ml de n-butanol. La phase butanolique est lavée par 2 fois 100ml d'eau distillée puis évaporée à sec sous pression réduite. Ce résidu est dissous dans 20ml de méthanol et la solution méthanolique est filtrée puis évaporée à sec. Le résidu est de 780mg soit un rendement de 0,39%. Le produit ainsi obtenu chromatographié sur plaque de gel de silice, révèle l'absence de substances susceptibles de migrer dans le solvant acétate d'éthyle, acide formique, eau (8-1-1 V/V), ce qui indique l'absence de substances de bas poids moléculaires (<10³). Cette technique permet de suivre la purification du produit. D'autre part, ce résidu dissous dans un alcool et traité à chaud par de l'acide chlorhydrique donne lieu à la formation d'une coloration rouge. Une chromatographie sur plaque contre un témoin révèle la formation de cyanidine, à l'exclusion d'autre anthocyane. La détermination du poids moléculaire de ce résidu en perméation de gel en milieu non aqueux révèle une répartition de poids moléculaires de 1000 à 5000 environ, avec la présence minoritaire de polymères de haut poids moléculaire (>5.10³).

### Exemple 2

### Extrait hydroéthanolique de rhizomes d'Alpinia galanga

### 2.1 - Extrait brut soluble

1kg de rhizomes frais d'Alpinia galanga contenant 88% d'eau sont broyés puis soumis à une première décoction d'une demi-heure dans 9,2 litres d'éthanol à 65,8% afin de réaliser en fait une décoction de l'équivalent de 220g de rhizomes secs par 10 litres d'éthanol à 60%. Puis les rhizomes sont décoctés deux fois de suite par 10 litres d'éthanol à 60% pendant une demi-heure. Les extraits sont réunis et évaporés à sec sous pression réduite. On reprend ensuite ce résidu par 220ml d'eau distillée à 20°C environ et on filtre. L'insoluble est repris par 220ml d'eau distillée à 20°C. Les filtrats sont réunis. Le résidu de cette solution aqueuse est de 38,4g soit un rendement de 3,84% par rapport aux rhizomes frais et de 17,45% par rapport aux rhizomes secs.

### 2.2 - Fraction procyanidolique

La solution aqueuse précédente est traitée de la même façon que dans le cas de l'exemple 1.2 jusqu'à l'évaporation de la phase butanolique. Le résidu est alors lavé à 20°C environ par deux fois 20ml d'eau distillée. Le résidu final est de 104mg soit un rendement de 0,01% par rapport aux rhizomes frais et de 0,4% par rapport aux rhizomes secs. Cette fraction présente les mêmes caractéristiques qualitatives que celles des cônes de Cupressus sempervirens. La mesure du poids moléculaire révèle un poids moyen de 4000.

### Exemple 3

### Fraction procyanidolique insoluble de Cupressus Sempervirens

200g de cônes secs concassés de Cupressus sempervirens sont décoctés trois fois de suite pendant une demi-heure par 2000ml de méthanol à 80%. Les extraits hydrométhanoliques sont réunis et évaporés à sec sous pression réduite. On lave ce résidu par deux fois 200ml d'eau distillée à 20°C environ. Les eaux de lavage sont éliminées. Le résidu est séché sous vide au dessicateur puis lavé à reflux par deux fois 200ml d'éther éthylique. La phasée éthérée est éliminée. Le résidu est ensuite lavé par deux fois 200ml d'acétate d'éthyle. La phase soluble dans l'acétate d'éthyle est éliminée. Puis le résidu est repris par 20ml de méthanol à 20°C environ. La solution méthanolique est filtrée. Le résidu de cette solution méthanolique est de 1,74g soit un rendement de 0,87%. Cette fraction insoluble dans l'eau présente les mêmes caractéristiques qualitatives que la fraction soluble obtenue à l'exemple 1. La détermination des poids moléculaires indique la présence très prépondérante de molécules de hauts poids moléculaires (5.10⁴).

Une étape supplémentaire peut être ajoutée après la dyafiltration finale au méthanol à 80%: on opère une autre dyafiltration avec de l'eau désionnisée afin d'éliminer totalement ou presque le méthanol. Cette opération se fait en réfrigérant le liquide en continu à +4°C. Une fois que le méthanol est éliminé, on concentre le plus possible la solution-suspension aqueuse de procyanidol polymère et on la lyophilise.

On peut aussi réaliser à ce stade une cristallisation à partir de la solution-suspension aqueuse. Le procyanidol polymère cristallise en aiguilles de couleur acajou foncé.

### Exemple 4

### Préparation d'une fraction de poids moléculaire 45000 daltons de procyanidols de cônes de Cupressus sempervirens

20kg de cônes de Cupressus sempervirens sont macérés 3 jours trois fois de suite dans 300 litres de méthanol à 80% (20% d'eau en volume). Les solutions extractives sont réunies pour former environ 850 litres.

La solution est ultrafiltrée sur des membranes tubulaires en polyéthersulfone présentant un seuil de coupure de 50000 daltons, afin d'éliminter les polymères très hautement polymérisés supérieurs à environ 5.10⁴.

Le filtrat est ensuite ultrafiltré sur des membranes de même type mais présentant un seuil de coupure de 20000 daltons. Le produit retenu sur ces membranes est lavé par diafiltration jusqu'à ce que les poids moléculaires faibles ne représentent plus que 1% du poids global de substance sèche.

### Exemple 5

### Préparation de proanthocyanidols de Cupressus sempervirens de poids moléculaire 3000

20 kg de cônes de Cupressus sempervirens sont mis à macérer trois fois successivement pendant 24 heures dans 300 litres de méthanol à 80 % à température ambiante.

Les filtrats sont réunis et ultrafiltrés sur une membrane en polyéthersulfone présentant un seuil de coupure de 5000 daltons vis-à-vis de protéines globulaires.

Le rétentat est ensuite dyafiltré par du méthanol à 80 % jusqu'à ce que le résidu sec du perméat soit inférieur à 0,1 %.

Le rétentat est ensuite ultrafiltré sur une membrane en polyéthersulfone présentant un seuil de coupure de 20000 vis-à-vis des protéines globulaires.

Ce dernier rétentat est dyafiltré par du méthanol à 80 % puis par du méthanol pur jusqu'à ce que le résidu sec du filtrat soit inférieur à 0,1 % et le titre de ce filtrat en méthanol supérieur à 97 %.

Le rétentat final est ensuite séché sous pression réduite à une température inférieure à 50°C. La quantité de proanthocyanidol obtenue est de 147g.

### Exemple 6

### Méthodes de dosage et détermination de la pureté

Le dosage de procyanidols obtenus à partir de Cupressus sempervirens peut faire appel à leur structure polyphénolique et donc à leur propriété réductrice de l'acide phosphotungstique (peu spécifique) ou à leur nature de proanthocyanidol c'est-à-dire à leur propriété de donner une anthocyane par traitement à chaud par les acides forts.

Dans les deux cas on obtient une valeur relative à un témoin plus ou moins éloigné de la molécule elle-même.

On peut aussi faire appel à la propriété tannante des procyanidols en solution aqueuse: il s'agit dans ce cas de déterminer la fixation sur de la poudre de peau préchromée.

### 1^{·}/ -Dosage de la fonction polyphénolique:

On chauffe à reflux 10g de tungstate de sodium avec 8ml d'acide orthophosphorique et 75ml d'eau désionnisée pendant 3 heures. On laisse refroidir et on complète à 100ml avec de l'eau désionnisée. Solution de carbonate de sodium à 15% dans de l'eau désionnisée: réactif B.

On pèse 50mg de procyanidol et on les dissout dans 10ml d'eau mesuré exactement.

On prélève 2ml de cette solution et on la dilue à 25ml dans une fiole jaugée, (solution C). Dans une fiole jaugée de 50ml, on place:
Solution C: 5 ml
Réactif A: 1ml
Réactif B: q.s.50ml.

On lit la D.O. à 715nm, 2 minutes exactement après le mélange du dernier réactif. On opère de même avec une gamme de pyrogallol (0 à 50mg) comme référence.

Le résultat pour le procyanidol polymère est de 43,5% de polyphénols totaux exprimés en pyrogallol.

### 2^{·}/ -Dosage de la fonction tannante:

Une solution de procyanidol préparée comme la solution C ci-dessus est agitée pendant une heure à température ambiante avec 1g de poudre de peau préchromée (Merck réf. 4332).

On opère ensuite un dosage de la fonction polyphénolique totale comme ci-dessus. La fonction tannante représente la différence entre la valeur trouvée pour les polyphénols totaux et la valeur trouvée après le traitement par la poudre de peau.

Le résultat pour le procyanidol polymère, est de 80% exprimé en fonction tannante.

### 3^{·}/ -Dosage de la fonction procyanidolique proprement dite:

10mg de procyanidol polymère mesurés environ exactement sont dissous dans 20ml d'éthanol à 75%.

On réalise un tube essai avec 1ml de cette solution, 5ml d'éthanol à 75% et 1ml d'acide chlorhydrique concentré. On place au bain marie bouillant pendant 30 minutes.

On réalise le tube témoin en mélangeant les mêmes constituants dans les mêmes proportions mais à la température ambiante et juste avant la lecture au spectrophotomètre.

On lit la D.O. de la solution essai à 540nm contre la solution témoin.

Le résultat pour le procyanidol polymère est de 0.60.

### 4^{·}/ -Détermination du poids moléculaire du procyanidol polymère:

On opère une chromatographie haute performance d'exclusion sur gel »Bondagel 125 (Waters) dans un solvant aprotique contre des témoins de polystyrène (Aldrich)
Débit: 0,5 ml/minute en isocratique.
Solvant: Chloroforme-diméthylsulfoxyde 50-50 (v/v)
Détection: D.O. à 283nm
Concentration des témoins: 1mg/ml dans le même solvant
Concentration du proanthocyanidol: 0,05mg/ml dans le même solvant
Poids moléculaires des témoins: 184.200, 50.000, 24.150, 7.025, 4.136 et 2.000.

Le témoin de 184.200 de poids moléculaire est utilisé comme témoin d'exclusion total sur le gel. En effet ce type de gel ne retient pas les poids moléculaires supérieurs à 50.000. On calcule ensuite le temps de rétention relatif de chaque échantillon par rapport au témoin totalement exclu.

On trace la courbe de correspondance entre le logarithme décimal du poids moléculaire et le temps relatif de rétention.

Le résultat pour le procyanidol polymère obtenu en appliquant la technique décrite précédemment est de 45.000.

### 5^{·}/ -Détermination de la pureté du procyanidol polymère par chromatographie sur gel:

La technique de chromatographie ci-dessus procure un tracé qui comprend deux pic. L'un correspond aux impuretés de poids moléculaire inférieurs à 5000. En effet par cette technique de chromatographie on constate que les poids moléculaires inférieur à 5000 sont rassemblés en un seul pic à la fin du chromatogramme.

La méthode de préparation qui fait intervenir une ultrafiltration finale sur une membrane au seuil de coupure de 20.000 environ permet d'obtenir à la fin de l'opération une solution concentrée de polymère retenu sur la membrane et une solution diluée de produits de poids moléculaires inférieurs à 20.000 et qui ne contient pas de procyanidol (vérifié par la méthode du 3^{·}).

On détermine alors la concentration en matière sèche totale des deux solutions. Puis on détermine leur absorption en U.V. à 283nm. On peut ainsi calculer le E% 1cm: Capacité d'absorption de la lumière du produit en solution à 1% sur 1cm de trajet optique de chaque produit.

On calcule alors sur le chromatogramme le rapport des aires des deux pics et on en conclut la pureté du procyanidol polymère.

Le résultat obtenu par cette méthode est de 98%.

### Exemple 7

### Essais antiviraux in vitro

Les essais antiviraux ont été réalisés sur le virus de l'herpès simplex de type I (HVS 1) implanté sur des fibroblastes de poumon embryonnaire humain.

On a déterminé sur un même lot de dilution virale correspondant à une dose infectante 50(DI 50)10^{5,7} virus par puits, l'efficacité antivirale des différents produits proanthocyanidoliques obtenus, c'est-à-dire la quantité de produit par puits, c'est-à-dire 100»l de milieu qui provoque une diminution de moitié de la DI 50.

A titre de comparaison, a été testée également la procyanidine A2, dimère procyanidolique extrait du marron d'Inde, de poids moléculaire 546.

| EXTRAIT | QUANTITE PROVOQUANT LA DIMINUTION DE MOITIE DE LA DI 50 |
|---|---|
| Procyanidols solubles de Cupressus sempervirens | 40 »g/ml |
| Procyanidols insolubles de Cupressus sempervirens | 72 »g/ml |
| Procyanidols solubles d'Alpinia galanga | 10 »g/ml |
| Procyanidine A2 | 156 »g/ml |

Un autre essai sur le virus HVS 1 a été réalisé avec une composition de proanthocyanidols de poids moléculaire 3000 telle que préparée dans l'exemple 5, dans les conditions suivantes :
- une suspension de cellules diploides humaines fibroblastiques (MRC5 Biomérieux) est diluée au 1/15 par du milieu MEM (Gilbco, ref. 041-02565) supplémenté avec 10 % de sérum de veau foetal, 1 % de solution de vitamines (Gibco, ref. 043-01120), 1 % de solution de glutamine 200 mM, 1 % de solution d'antibiotiques (pénicilline à 200 U/ml, streptomycine à 50 »g/ml, amphotéricine B à 2 »g/ml). Cette suspension est implantée à raison de 100»l par puits sur des plaques de 96 puits. On incube 48 heures à 37°C sous une atmosphère contenant 5 % de CO₂.
- On réalise une solution mère à 0,5 % de proanthocyanidols correspondant à la définit)on ci-dessus. On prépare ensuite des dilutions successives de raison 2 jusqu'au 1/512 de cette solution mère dans le même milieu que ci-dessus mais contenant 2 % de sérum de veau foetal. On élimine les dilutions inférieures au 1/16.
- La suspension de HSV 1, typée, congelée et titrée est diluée de 10 en 10 jusqu'à 10⁻⁷ dans le milieu ci-dessus mais ne contenant que 2 % de sérum de veau foetal. On répartit dans différents tubes 1 ml de chaque dilution auquel on ajoute pour les essais 1 ml de chaque dilution de proanthocyanidols, et pour les témoins 1 ml de milieu contenant 2 % de sérum de veau foetal. On laisse en contact 1 heure à 37°C.
- On élimine le surnageant des puits où sont cultivées les cellules et on place dans chaque puits 200 »l de chaque dilution.
- On laisse incuber 1 heure à 37°C avec 5 % de CO₂. On rince deux fois par 200 »l de milieu avec 2 % de sérum de veau foetal puis on ajoute 200 »l du même milieu et on laisse incuber 5 jours dans les mêmes conditions atmosphériques.
- On lit ensuite les effets cytopathogènes dans chaque puits de manière directe.

On a observé que la réduction de 50 % du titre viral est obtenue pour une concentration d'environ 25 »g/ml.

### Exemple 8

### Essai des procyanidols de Cupressus sempervirens solubles dans l'eau sur le virus du syndrome immunodéficitaire acquis humain (HIV-1).

### a/ Effet antiviral

L'essai est réalisé sur des cellules H9 infectées par des dilutions décimales successives de HIV-1 pendant 90 minutes.

Après infection des cellules, celles-ci sont traitées par des concentrations décroissantes de procyanidols à des doses inférieures à la cytotoxicité constatée sur cette lignée cellulaire (150»g/ml).

Après 10 jours de culture, les cellules sont centrifugées et les virus sont recherchés par un examen radio-immunologique (recherche de la protéine p24) sur le surnageant.

### Résultats:

Les procyanidols solubles du cyprès obtenus selon l'exemple 1 procurent une inhibition de l'expression virale de 97% à la concentration de 50»g/ml.

### b/ Effet virucide

La suspension virale (HIV1) est traitée pendant 30 minutes à 37°C par des concentrations décroissantes de procyanidols solubles du cyprès. Puis les cellules H9 sont infectées par des dilutions décimales successives de la suspension précédente.

### Résultats:

Les procyanidols solubles du cyprès procurent une réduction du titre viral de 1,5 log (titre initial: 3.10⁴) à la concentration de 50»g/ml, et une réduction de 2,5 log à la concentration de 150»g/ml.

### Exemple 9

### Essai des procyanidols solubles dans l'eau de Cupressus sempervirens sur le virus de l'herpès Simplex de type II.

Les essais antiviraux ont été réalisés sur le virus de l'herpès simplex type II implanté sur des fibroblastes de poumon embryonnaire humain.

On a déterminé sur un lot de suspension virale correspondant à une dose infectante 50(DI 50)2,5.10⁴ virus par puits, l'efficacité antivirale des procyanidols solubles de Cupressus sempervirens, c'est-à-dire la quantité de produit par puits (c'est-à-dire dans 100»l de milieu) qui provoque une diminution de moitié de la DI50.

La valeur trouvée est de 3,3»g/100»l.

### Exemple 10

### Essai des procyanidols solubles dans l'eau de Cupressus sempervirens sur le virus Varicelle-Zona

Les essais antiviraux ont été réalisés sur le virus de la varicelle et du zona implanté sur des fibroblastes de poumon embryonnaire humain.

On a déterminé un lot de suspension virale correspondant à une dose infectante 50 (DI50) 5.10³ virus par puits, l'efficacité anti-virale des procyanidols solubles de Cupressus sempervirens, c'est-à-dire la quantité de produit par puits (c'est-à-dire 100»l de milieu) qui provoque une diminution de moitié de la DI50.

La valeur trouvée est de 6»g/100»l.

Un deuxième test, réalisé avec une composition de proanthocyanidols de poids moléculaire 3000 a montré que le pouvoir virucide de cette composition est de 100 % pour un titre viral de départ de 3.10³, et pour une concentration de 312,5 »g/ml dans les conditions opératoires détaillées données pour le deuxième test décrit pour le virus HVS 1 dans l'exemple 5.

### Exemple 11

### Essai des procyanidols solubles dans l'eau de Cupressus sempervirens sur le virus Influenza A

Les essais antiviraux ont été réalisés sur le virus Influenza A implanté sur des cellules d'adénocarinome de colon humain.

On a déterminé sur un lot de suspension virale correspondant à une dose infectante 50 (DI50) 7.10³ virus par puits, l'efficacité antivirale des procyanidols solubles de Cupressus sempervirens, c'est-à-dire la quantité de produit par puits (c'est-à-dire dans 100»l de milieu) qui provoque une diminution de moitié de la DI50.

La valeur trouvée est de 12»g/100»l.

Un deuxième test est réalisé dans les conditions suivantes :
- Une suspension de cellules d'adénocarcinome de colon humain (HT 29 Bimérieux) est diluée au 1/15 par du milieu MEM (Gibco, ref. 041-02565) supplémenté avec 10 % de sérum de veau foetal, 1 % de solution de vitamines (Gibco, ref. 043-01120,), 1 % de glutamine 200 mM, 1 % de solution d'acides aminés non essentiels (Gibco, ref. 043-01140), 0,1 % de solution de transférine humaine à 1 mg/100 ml, 1 % de solution d'antibiotiques (pénicilline à 200 U/ml, streptomycine à 50 »g/ml, amphotéricine B à 2 »g/ml). Cette suspension est implantée à raison de 100 »l par puits sur des plaques de 96 puits. On incube 48 heures à 37°C sous une atmosphère contenant 5 % de CO₂.
- On réalise une solution mère à 0,5 % de proanthocyanidols de composition de poids moléculaire 3000. On prépare ensuite des dilutions successives de raison 2 jusqu'au 1/16 de cette solution mère dans le même milieu que ci-dessus mais contenant 2 % de sérum de veau foetal.
- La suspension de virus influenza A, typée, congelée et titrée est diluée de 10 en 10 jusqu'à 10⁻⁷ dans le milieu ci-dessus mais ne contenant que 2 % de sérum de veau foetal. On répartit dans différents tubes 1 ml de chaque dilution auquel on ajoute pour les essais 1 ml de la dilution de proanthocyanidols, et pour les témoins 1 ml de milieu contenant 2 % de sérum de veau foetal. On laisse en contact 1 heure à 37°C. On élimine le surnageant des puits où sont cultivées les cellules et on place dans chaque puits 200 ml de chaque dilution.
- On laisse incuber 1 heure à 37°C et 5 % de CO₂. On rince deux fois par 200 »l de milieu avec 2 % de sérum de veau foetal, puis on ajoute 200 »l du même milieu et on laisse incuber 24 heures dans les mêmes conditions atmosphériques.
- La lecture des résultats se fait par une technique d'immunoflorescence.
- On congèle la plaque à -20°C pendant au moins 1/2 heure après avoir éliminé le milieu et on fixe les cellules par 100 »l d'éthanol à 96 %. On rince trois fois avec 100 »l de tampon PBS et on ajoute 100 »l de sérum antigrippe A de lapin dilué au 1/100 dans du tampon PBS. On laisse incuber 30 à 40 minutes à 37°C. on rince trois fois 100 »l de tampon PBS et on ajoute ensuite 100 »l d'une solution au 1/100 d'anticorps de lapin marqués à la fluoresceine anti IgG (H + L).
- On laisse agir 45 minutes à 37°C, on rince par trois fois 100 »l de tampon PBS. On compte ensuite le nombre de celllules fluorescentes par puits.

Dans ces conditions, le pouvoir virucide des proanthocyanidols utilisés, est de 85 %, pour un titre viral de départ de 5.10³, et pour une concentration de 312,5 »/ml.

### Exemple 12

### Essai des procyanidols solubles dans l'eau de Cupressus sempervirens sur le virus ECHO 25

Les essais antiviraux ont été réalisés sur le virus ECHO 25 implanté sur des fibroblastes de poumon embryonnaire humain.

On a déterminé sur un lot de suspension virale correspondant à une dose infectante 50 (DI50) 10⁴ virus par puits, l'efficacité antivirale des procyanidols solubles de Cupressus sempervirens, c'est-à-dire la quantité de produit par puits (c'est-à-dire dans 100»l de milieu) qui provoque une diminution de moitié de la DI50.

La valeur trouvée est de 31»g/100»l.

### Exemple 13

### Effet d'une composition de proanthocyanidols sur le cytomégalovirus.

Une suspension de cellules diploïdes humaines fibroblastiques (MRC5 Biomérieux) est diluée au 1/15 par du milieu MEM (Gibco, ref. 041-02565), supplémenté avec 10 % de sérum de veau foetal, 1 % de solution de vitamines (Gibco, ref. 043-01120), 1 % de solution de glutamine 200 mM, 1 % de solution d'antibiotiques (pénicilline à 200 U/ml, streptomycine à 50 »g/ml, amphotéricine B à 2 »g/ml). Cette suspension est implantée à raison de 100 »l par puits sur ces plaques de 96 puits. On incube 48 heures à 37°C sous une atmosphère contenant 5 % de CO₂.

On réalise une solution mère à 0,5 % de proanthocyanidols de poids molécualire 3000. On prépare ensuite des dilutions successives de raison 2 jusqu'au 1/16 de cette solution mère dans le même milieu que ci-dessus mais contenant 2 % de sérum de veau foetal.

La suspension de CMV, typée, congelée et titrée est diluée au 1/10 puis de 2 en 2 jusqu'à 10⁻² dans le milieu ci-dessus mais ne contenant que 2 % de sérum de veau foetal. On répartit dans différents tubes 1 ml de chaque dilution auquel on ajoute pour les essais 1 ml de chaque dilution de proanthocyanidols ayant la composition ci-dessus, et pour les témoins 1 ml de milieu contenant 2 % de sérum de veau foetal. On laisse en contact 1 heure à 37°C.

On élimine le surnageant des puits où sont cultivées les cellules et on place dans chaque puits 200 »l de chaque dilution.

On laisse incuber 2 heures à 37°C avec 5 % de CO₂. On rince deux fois par 200 »l de milieu avec 2 % de sérum de veau foetal, puis on ajoute 200 »l de même milieu et on laisse incuber 12 heures dans les mêmes conditions atmosphériques.

On lit ensuite les effet cytophatogènes dans chaque puits par une technique ELISA.

Dans ces conditions, le pouvoir virucide des proanthocyanidols utilisés est de 100 % pour titre viral de départ de 5,65.10² et pour une concentration de 312,5 »/ml.

### Exemple 14

### Activité de la fraction procyanidolique de poids moléculaire 45000 sur les Rotavirus

Les essais antiviraux ont été réalisés sur une souche sauvage de rotavirus implantée sur des cellules de reins embryonnaires de singes rhésus MA104.

On a déterminé sur un lot de suspension virale correspondant à une dose infectante 50 (DI50) 2.10³ virus par puits, l'efficacité antivirale de cette fraction procyanidolique, c'est-à-dire la quantité du produit par puits (c'est-à-dire dans 100»l de milieu) qui provoque une diminution de moitié de la DI50. La valeur trouvée est de 8»g/100»l.

### Exemple 15

### Essai de proanthocyanidols de poids moléculaire 3000 sur le virus de l'hépatite A

Le virus de l'hépatite A est cultivé sur des hépatomes maintenus en survie à 32°C. Les procyanidols sont dissous directement dans le milieu de culture.

On constate que la production antigénique est inhibée de 20 % pour une concentration en procyanidols de 5 »g/ml, de 34 % pour une concentration de 10 »g/ml et de 96 % pour une concentration de 20 »g/ml.

### Exemple 16

### Effet de proanthocyanidols de poids moléculaires 3000 sur l'action d'une collagènase

On prépare un gel d'agarose mélangé à du collagène acido-soluble de peau de bovin.

Ce gel est coulé dans des boites de Pétri. Dans des puitss creusés à l'emporte pièce dans le gel, on dépose des concentrations croissantes d'une solution de collagènase. L'enzyme diffuse autour du puitss et dégrade le collagène en fonction de la concentration de l'enzyme.

Après coloration des boites au rouge Sirius, on distingue en jaune clair des zones de collagénolyse sur un fond rouge de collagène non dégradé. Le diamètre des plages de lyse est proportionnel à la concentration d'enzyme déposée dans un puits donné. On incube d'abord le collagène avec une solution de proanthocyanidol préparée comme ci-dessus, à des concentrations allant de 1 % à 5 %, et l'on prépare des boites de Pétri comme pour les témoins. On constate alors que les plages de lyse ont diminué de façon significative.

### Exemple 17

### Effets de proanthocyanidols de poids moléculaire 3000 sur l'action d'une élastase

Les proanthocyanidols sont préparés comme dans l'exemple 5.

On opère également pour le test comme pour l'exemple 16, mais en remplaçant le collagène par de l'élastine fibreuse micronisée et la collagènase par de l'élastase.

L'élastine fibreuse rend le gel opaque et une coloration n'est pas nécessaire.

On constate que lorsqu'une boite est préparée avec de l'élastine prélablement incubée avec les proanthocyanidols, les plages de lyses sont réduites de façon significative.

### Exemple 18

### Effet de proanthocyanidols de poids moléculaire 3000 sur une collagènase appliquée "ex vivo"

Les procyanidols sont préparés comme pour l'exemple 5.

On colore le collagène d'une coupe de peau humaine par le picro-indigocarmin. Les coupes témoins montrent que le collagène est dans un état normal.

On expose certaines de ces coupes à l'action d'une collagènase et l'on observe ensuite la dégradation partielle du collagène.

D'autres lames sont préalablement incubées avec les procyanidols et ensuite soumises à l'action de la collagènase. On constate une réduction nette de la dégradation du collagène dans ces coupes par rapport aux coupes témoins.

### Exemple 19

### Effet de proanthocyanidols de poids moléculaire 3000 sur une élastase appliquée "ex vivo"

On opère comme pour l'exemple 18, mais l'on colore l'élastine avec la fuschine-(+)catéchine, et l'enzyme utilisée est ici une élastase. On constate comme pour l'exemple 18, une réduction de l'attaque de l'enzyme sur son substrat.

### Exemple 20

### Stimulation de la fibrillogénèse du collagène par des proanthocyanidols de poids moléculaire 3000

Les procyanidols sont préparés comme dans l'exemple 19.

Du collagène acido-soluble de peau bovine est mélangé à du gel d'agarose. Ce collagène contient surtout des molécules isolées qui ont une faible tendance à l'agrégation. Cette agrégation peut être observée après coloration du collagène au rouge Sirius au microscope optique, et quantifiée par analyse d'image.

On incube préalablement le collagène avec les procyanidols et l'on constate alors la formation d'agrégats de taille nettement plus grande. La surface occupée par ces agrégats est significativement plus grande que celle des témoins.

Afin de vérifier que ces agrégats ne sont pas de simples précipités amorphes, on examine les agrégats formés au microscope électronique. On constate alors que le collagène traité présente des formations de fibres régulières et portant la striation caractéristique du type interstitiel.

### Exemple 21

### Stimulation de l'attachement des fibres élastiques aux fibroblastes par des proanthocyanidols polymères de poids moléculaire 3000.

Les procyanidols sont préparés comme dans l'exemple 19.

Des fibroblastes de peau humaine sont cultivés sur milieu DMEM additionné de 10 % de sérum de veau foetal, et de procyanidols aux concentrations 1-0,1-0,01-0,001 mg/ml.

Une culture ne reçoit pas de produit et est utilisée comme témoin.

On ajoute aux cultures à confluence de l'élastine fibreuse micronisée à la concentration de 0,2 mg/ml et on laisse incuber 48 heures dans les conditions de la culture.

A la fin de l'incubation, les cellules sont rincées et l'élastine colorée selon la méthode de Verhoeff modifiée par Godeau ("Selective staining technique for the identification of human skin elastic fiber" Pathol. Biol., (1984), **32**, p.215-216).

On évalue alors quantitativement l'élastine fixée aux cellules par analyse d'image :

| Concentration en procyanidols | Aire de l'élastine fixées |
|---|---|
| 0 mg/ml | 3114 »² |
| 0,001 mg/ml | 5059 »² |
| 0,01 mg/ml | 5789 »² |
| 0,1 mg/ml | 5227 »² |
| 1 mg/ml | 3484 »² |

On constate que la présence de procyanidols dans les milieux de culture des fibroblastes confluents augmente la quantité d'élastine attachée aux cellules. La concentration optimale est de 0,01 mg/ml.

### Exemple 22

### Effet des procyanidols de poids moléculaire 3000 sur la perméabilité vasculaire centrale et périphérique

Les essais ont été réalisés sur 4 lots de 5 rats blancs Witsar d'un poids moyen initial de 250 à 300 g chacun.

Le principe du test consiste à augmenter artificiellement par administration intraveineuse de collagènase, la perméabilité vasculaire à la fois dans les grands troncs et dans les capillaires et à noter l'effet protecteur du produit administré quotidiennement par voie orale pendant trois semaines. Cette administration de protéase reproduit un mécanisme pathologique observé dans les états inflammatoires, dans le diabète, dans l'athérosclérose et aussi dans le vieillissement. Cet état peut aussi être observé dans des cas d'infection virale lors de la lyse du tissu conjonctif à la suite de la destruction cellulaire opérée par le virus.
- Lot 1 :: **Témoins négatifs** : ces rats reçoivent à la place du traitement du sérum physiologique par voie orale et à la fin du traitement, une injection intraveineuse de sérum physiologique et une demi-heure après, une injection intraveineuse de traceur de perméabilité (peroxydase du raifort de type VI Sigma à raison de 100 mg/kg.
- Lot 2 :: **Témoins positifs** : ces rats reçoivent également du sérum physiologique par voie orale à la place du traitement mais à la fin de celui-ci, ils reçoivent de la collagènase par voie intraveineuse (270 unité de collagènase bactérienne) puis 30 minutes après le traceur de perméabilité. Les résultats de ce lot comparés à ceux du lot 1 permettent d'évaluer l'effet perméabilisant de la protéase administrée.
- Lot 3 :: **Effet du produit sans collagènase** : les rats reçoivent une administration per orale quotidienne pendant 21 jours de procyanidols à la dose de 50 mg/kg dans du sérum physiologique. A la fin du traitement, ils reçoivent une injection intraveineuse de sérum physiologique et 30 minutes après le traceur de perméabilité par la même voie. Les résultats de ce lot doivent montrer si le produit induit ou non une altération de la perméabilité vasculaire.
- Lot 4 :: **Lot expérimental proprement dit** : les rats reçoivent les 21 administrations de procyanidols comme le lot 3. Mais à la fin du traitement, ils reçoivent une injection de collagènase, puis 30 minutes après le traceur de perméabilité. Les résultats de ce lot comparés à ceux du lot 2 montrent si le traitement a réduit ou non, l'effet perméabilisant de la collagènase.

Tous les rats sont ensuite sacrifiés et des coupes histologiques de certains de leurs tissus sont réalisées.

On constate qu'aucune altération de la perméabilité vasculaire normale n'intervient sous l'effet du traitement des rats pendant un mois avec le produit étudié.

Il ressort de cette étude, que les procyanidols exercent une action très nette sur les parois des gros vaisseaux et aussi sur les petits vaisseaux et les capillaires du cerveau (barrière hémato-encéphalique).

Au niveau de l'aorte la neutralisation des effets de la collagènase a été considérable mais il est encore plus intense au niveau de la veine cave et du lobe frontal du cerveau. Dans ces deux organes, les conditions de perméabilité ont été maintenues à la normale malgré l'injection de collagènase.

### Exemple 23

### Effet des procyanidols de poids moléculaire 3000 sur le lathyrisme expérimental de la souris

Lors de certaines pathologies le tissu conjonctif subit des lésions et des modifications de structure. Dans le but d'observer l'action des proanthocyanidols de poids moléculaire 3000 sur le tissu conjonctif, nous avons traité des souris avec un agent connu pour induire un lathyrisme expérimental : le β-aminopropionitrile (BAPN).

Les souris sont réparties en trois lots :
- Lot 1 :: **lot témoin négatif** : ces souris reçoivent quotidiennement 100ml/kg de sérum physiologique par voie intrapéritonéale pendant 11 semaines.
- Lot 2 :: **Lot témoin positif** : ces souris reçoivent quotidiennement pendant 11 semaines, 1000 mg/kg de β-aminopropionotrile (BAPN), produit connu pour induire un lathyrisme, dissous à raison de 100 mg/ml dans du sérum physiologique à raison de 100 mg/ml pou le BAPN et de 5 mg/ml pour les procyanidols.
- Lot 3 :: **Lot expérimental** : les souris reçoivent quotidiennement pendant 11 semaines 1000 mg/kg de BAPN et 50 mg/kg de procyanidols comme dans l'exemple 18. Les deux produits sont dissous dans du sérum physiologique à raison de 100 mg/ml pour le BAPN et de 5 mg/ml pour les procyanidols.

A la fin du traitement, les souris sont sacrifiées et l'on prélève la peau de l'aorte.

Les organes sont ensuite fixés pour examen histologique.

On constate au niveau de l'aorte, une désorganisation profonde des lames élastiques dans le lot 2 par rapport au lot 1. Le lot 3 apparait tout à fait similaire au lot 1 et l'on observe donc l'effet protecteur des proanthocyanidols vis-à-vis du lathyrisme induit par le BAPN.

Au niveau de la peau, on observe que le lot 2 présente des trousseaux de collagène positionnés de manière anarchique ce qui n'est pas le cas du lot 1. Le lot 3 présente une meilleur organisation des trousseaux de collagène.

### Exemple 24

### Stimulation de la synthèse des protéines d'hépatomes par des proanthocyanidols de poids moléculaires 3000

Les procyanidols sont préparés comme dans l'exemple 19.

Le milieu de culture d'hépatomes maintenus en survie à 32°C est additionné de quantités croissantes de procyanidols. On mesure l'influence de la concentration de ce produit sur la synthèse des protéines de cette lignée cellulaire continue.

On constate que 10 »g/ml de procyanidols augmente la synthèse protéique de 130 %, 40 »g/ml l'augmente de 180 % et 160 »g/ml de 250 %.

### Exemple 25

### Essais de toxicité sur l'animal des proanthocyanidols obtenus dans les exemples précédents

La toxicité par voie orale de tous les proanthocyanidols obtenus, recherchée chez la souris n'a pu être évaluée, les doses à ingérer dépassant les possibilités des animaux et étant supérieures à 2g par kg de poids corporel.

Les tests d'irritation primaire cutanée des mêmes produits ainsi que les tests d'irritation oculaire pratiqués chez le lapin en solution aqueuse à 5% pour les fractions solubles ont permis de définir les produits comme non irritants. Les tests d'irritation primaire cutanée des protanthocyanidols insolubles en solution à 5% dans un mélange à 50% de monopropylèneglycol et d'eau ont permis de définir ces produits comme non irritants comparativement à un mélange pur de monopropylèneglycol et d'eau à 50%.

### Exemple 26

### Essais cliniques sur les procyanidols d'Alpinia galanga

Les essais cliniques ont été réalisés sur les proanthocyanidols d'Alpinia galanga obtenus à l'exemple 2.

L'étude a porté sur 140 malades, présentant des symptômes herpétiques débutant depuis moins de 24 heures, répartis comme suit:
.24 femmes d'âges compris entre 17 et 47 ans présentant un herpès récurrant lié au cycle menstruel. Parmi elles, on comptait 18 cas d'herpès labial et 6 cas d'herpès touchant des régions diverses du corps non muqueuses (ailes du nez, fesses, joues).

116 hommes d'âges compris entre 19 et 54 ans présentant tous un herpès labial.

Les femmes ont été réparties en quatre groupes:
. un groupe témoin d'herpès labial récurrant,
. un groupe essai de la même affection soit à chaque fois 9 personnes,
. un groupe témoin d'herpès divers,
. un groupe essai d'herpès divers, soit à chaque fois 3 personnes.

Les hommes ont été répartis en deux groupes égaux de 58 personnes, un groupe essai et un groupe témoin aussi homogène que possible.

Les témoins sont traités jusqu'à disparition des lésions à raison d'une application à 7h, 9h, 11h, 13h, 15h, 17h et 19h pendant trois jours puis d'une application matin, midi et soir jusqu'à la fin, d'un gel placebo de composition suivante:
Polyacrylate de sodium réticulé: 0,75%
Caramel: qs pour coloration
Conservateurs: paraoxybenzoate de propyle et de méthyle: 0,35%.
Eau distillée: qs 100%.
Les groupes essais sont traités dans les mêmes conditions que les témoins avec un gel de la composition suivante:
Polyacrylate de sodium réticulé: 0,75%
Fraction de proanthocyanidols: 2%
Conservateurs: paraoxybenzoate de propyle et de méthyle: 0,35%.
Eau distillée: qs 100%.

### Résultats

On apprécie le temps que met la lésion à disparaître complètement ne laissant plus qu'une trace cicatricielle totalement fermée et sèche.
Groupe témoin des hommes: 5ème jour: 1
6ème jour: 4 et 1 abandon
7ème jour: 4
8ème jour: 16 et 2 abandons
9ème jour: 11
10ème jour: 14
11ème jour: 2
12ème jour: 3
Groupe essai des hommes: 1er jour: 1
2ème jour: 5
3ème jour: 37
4ème jour: 12
5ème jour: 1
6ème jour: 0
7ème jour: 1 abandon
8ème jour: 1
Premier groupe témoin des femmes:
5ème jour: 1
6ème jour: 1
7ème jour: 4
8ème jour: 0
9ème jour: 2 abandons
10ème au 15ème jour: 0
16ème jour: 1
Premier groupe essai des femmes:
1er jour: 4
2ème jour: 2
3ème jour: 2
4ème jour: 1
Deuxième groupe témoin des femmes:
13ème jour: 2
17ème jour: 1
Deuxième groupe essai des femmes:
4ème jour: 3
Outre une efficacité importante du produit, on note chez tous les sujets traités une excellente tolérance au traitement, aucun effet secondaire n'ayant été signalé, les abandons en cours d'essai ne sont pas liés à une intolérance au produit.

## Revendications

1. Composition à base de proanthocyanidols, notamment d'origine végétale, caractérisée par une proportion pondérale d'au moins 50% de molécules ayant des poids moléculaires apparents supérieurs à 2000, cette proportion étant constituée d'au moins 40% de proanthocyanidols, ce pourcentage étant exprimé en fonction tannante, et en ce que ladite composition est essentiellement exempte:
- d'essences aromatiques et notamment d'huiles essentielles, de résines et autres substances lipophiles
- de sucres polymérisés tels que l'amidon, les gommes, les dérivés cellulosiques,
- de sucres oligomères tels que le saccharose, le glucose, le levulose.

2. Composition à base de proanthocyanidols selon la revendication 1, caractérisée en ce qu'elle est essentiellement exempte:
- de protéines végétales,
- d'acide gallique et de tanins galliques,
- de flavonoïdes n'appartenant pas à la famille des flavanes-diols,
- de flavanes-diols oligomères,
- d'acides phénols.

3. Composition selon la revendication 1 ou la revendication 2, caractérisée en ce qu'elle est constituée d'au moins 95% en poids sec de proanthocyanidols.

4. Composition selon l'une quelconque des revendications 1 à 3, caractérisée en ce que les proanthocyanidols ont un poids moléculaire compris entre environ 2000 ± 500 et environ 55000 ± 5000.

5. Composition selon l'une quelconque des revendications 1 à 4, caractérisée en ce qu'elle comprend des proanthocyanidols de poids moléculaire environ 45000 ± 5000.

6. Composition selon l'une quelconque des revendications 1 à 4 caractérisée en ce qu'elle comprend des proanthocyanidols de poids moléculaire environ 5000 ± 500.

7. Composition selon l'une quelconque des revendications 1 à 4, caractérisée en ce qu'elle comprend des proanthocyanidols de poids moléculaire environ 3000 ± 300.

8. Composition selon l'une quelconque des revendications 1 à 7, caractérisée en ce qu'elles comprennent des proanthocyanidols sous forme de polymères répondant à la formule suivante: 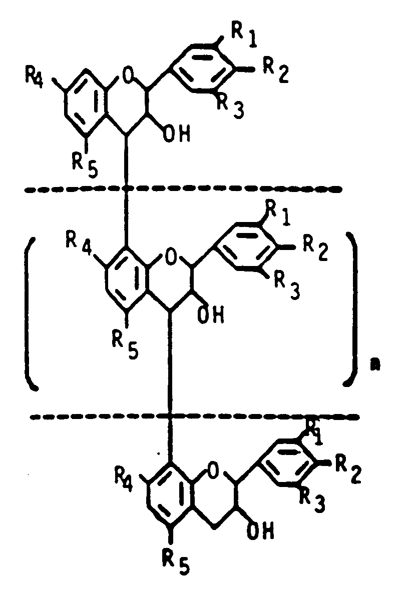 dans laquelle n est un entier supérieur ou égal à 3 et de préférence compris entre 3 et 350.
R₁, R₂, R₃, R₄ et R₅ sont ensemble ou séparément, H, OH ou OCH₃, et de préférence en ce qu'il s'agit d'un polymère de procyanidol, dans lequel R₁, R₂, R₄ et R₅ sont des groupements hydroxyle et R₃ de l'hydrogène, ou en ce qu'il s'agit de préférence un polymère de prodelphinidol dans lequel R₁, R₂, R₃, R₄ et R₅ sont des groupements hydroxyle ou en ce qu'il s'agit d'un polymère de promalvidol dans lequel R₁ et R₃ sont des groupements méthoxy et R₂, R₄ et R₅ sont des groupements hydroxyles.

9. Extrait végétal selon l'une quelconque des revendications 1 à 8, tel qu'obtenu à partir d'un végétal choisi parmi les conifères tels que Cupressus sempervirens, Pinus maritima, les acacias, les mimosacées.

10. Procédé pour la préparation d'une composition à base de proanthocyanidols à partir d'une partie de plante contenant des proanthocyanidols de PM supérieur à 2000 selon l'une quelconque des revendications 1 à 9, caractérisé en ce qu'il comprend les étapes suivantes:
- décoction ou macération dans un alcool, notamment le méthanol ou l'éthanol, pour éliminer les polysaccharides notamment l'amidon.
- récupération des extraits hydroalcooliques et évaporation à sec, sous pression réduite, suivie éventuellement de lavage et récupération du résidu sec,
- reprise du résidu sec et filtration pour éliminer une partie de la fraction aromatique de l'extrait,
- extraction à contre-courant, à l'aide successivement d'au moins 3 solvants de polarité croissante, notamment l'éther éthylique, l'acétate d'éthyle, le butanol ou le méthanol notamment pour éliminer les essences aromatiques restant, la plus grande partie possible des oligomères de PM inférieur à 2000 et des monomères de proanthocyanidols et pour récupérer dans la phase alcoolique un extrait végétal contenant les proanthocyanidols de poids moléculaire supérieur à 2000,
- le cas échéant, la purification de l'extrait obtenu pour éliminer les solvants restants.

11. Procédé pour la préparation d'une composition à base de proanthocyanidols à partir d'une partie de plante contenant des proanthocyanidols de PM supérieur à 2000 selon l'une quelconque des revendications 1 à 9, caractérisé en ce qu'il comprend les étapes suivantes:
- décoction ou macération dans un alcool, notamment le méthanol ou l'éthanol, pour éliminer les polysaccharides notamment l'amidon,
- ultrafiltration des solutions hydroalcooliques sur une membrane organique d'une porosité telle que son seuil de coupure serait de 20000 Daltons pour une protéine globulaire ultrafiltrée dans une solution aqueuse,
- récupération des extraits hydroalcooliques et évaporation à sec, sous pression réduite, suivie éventuellement de lavage et récupération du résidu sec,

12. Procédé selon la revendication 10 ou 11, caractérisé en ce que l'extrait végétal récupéré est concentré puis lyophilisé ou cristallisé.

13. Procédé selon la revendication 10 ou la revendication 11, caractérisé en ce que la matière végétale initiale est constituée par des cônes de Cupressus sempervirens.

14. Composition selon l'une quelconque des revendications 1 à 9, caractérisée en ce qu'elle est non cytotoxique.

15. Composition pharmaceutique, caractérisée en ce qu'elle comprend à titre de principe actif, une quantité efficace d'au moins une composition de proanthocyanidols selon l'une quelconque des revendications 1 à 9, en association avec un véhicule pharmaceutique acceptable.

16. Utilisation d'une composition de proanthocyanidols selon l'une quelconque des revendications 1 à 9, pour la fabrication d'un médicament à activité anti-virale.

17. Utilisation d'une composition de proanthocyanidols selon l'une quelconque des revendications 1 à 3, pour la fabrication d'un médicament à activité anti-protéase.

## Claims

1. A proanthocyanidol-based composition, especially of plant origin, characterised by a proportion by weight of at least 50% of molecules having apparent molecular weights greater than 2000, this proportion being composed of at least 40% of proanthocyanidols, this percentage being expressed by its tanning function, and in that said composition is substantially free from:
- aromatic spirits and particularly essential oils, resins and other lipophilic substances
- polymerized sugars, such as starch, gums, cellulose derivatives
- oligomeric sugars such as sucrose, glucose, fructose.

2. A proanthocyanidol-based composition according to Claim 1, characterised in that it is substantially free from:
- vegetable proteins,
- gallic acid and gallic tannins,
- flavonoids not belonging to the family of flavan-diols
- oligomeric flavan-diols
- phenol acids.

3. A composition according to Claim 1 or Claim 2, characterised in that it is composed of at least 95% dry weight of proanthocyanidols.

4. A composition according to any one of Claims 1 to 3, characterised in that proanthocyanidols have a molecular weight between approximately 2000 ± 500 and approximately 55000 ± 5000.

5. A composition according to any one of Claims 1 to 4, characterised in that it comprises proanthocyanidols of molecular weight of approximately 45000 ± 5000.

6. A composition according to any one of Claims 1 to 4, characterised in that it comprises proanthocyanidols of molecular weight of approximately 5000 ± 500.

7. A composition according to any one of Claims 1 to 4, characterised in that it comprises proanthocyanidols of molecular weight of approximately 3000 ± 300.

8. A composition according to any one of Claims 1 to 7, characterised in that it comprises proanthocyanidols in the form of polymers corresponding to the following formula: 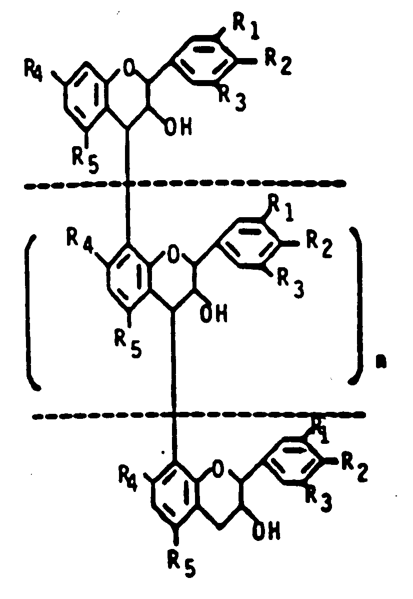 in which n is a whole number greater than or equal to 3, preferably between 3 and 350.
R₁, R₂, R₃, R₄ and R₅ are together or separately, H, OH or OCH₃, and preferably in that there is a proanthocyanidol polymer, in which R₁, R₂, R₄ and R₅ are hydroxyl groups and R₃ is hydrogen, or in that there is preferably a prodelphinidol polymer in which R₁, R₂, R₃, R₄ and R₅ are hydroxyl groups or in that there is a promalvidol polymer in which R₁ and R₃ are methoxy groups and R₂, R₄ and R₅ are hydroxyl groups.

9. A plant extract according to any one of Claims 1 to 8, such as obtained from a plant chosen from conifers such as Cupressus sempervirens, Pinus maritima, the acacias, the mimosas.

10. A process for the preparation of a proanthocyanidol-based composition from a part of a plant containing proanthocyanidols with molecular weights greater than 2000 according to any one of Claims 1 to 9, characterised in that it comprises the following steps:
- decoction or maceration in an alcohol, in particular methanol or ethanol, in order to remove polysaccharides, particularly starch.
- recuperation of the hydroalcoholic extract and evaporation to dryness, under reduced pressure, followed possibly by washing and recuperation of the dry residue,
- taking up the dry residue and filtering it to remove part of the aromatic fraction of the extract,
- countercurrent extraction, with the aid of at least 3 solvents of increasing polarity successively, particularly diethyl ether, ethyl acetate, butanol or methanol in particular to remove any remaining aromatic spirits, the greatest possible part of oligomers with MW less than 2000 and proanthocyanidol monomers and in order to recover in the alcohol phase a plant extract containing the proanthocyanidols with molecular weights greater than 2000.
- if the need arises, purification of the extract obtained in order to remove residual solvents.

11. A process for the preparation of a proanthocyanidol-based composition from a part of a plant containing proanthocyanidols with molecular weights greater than 2000, according to any one of Claims 1 to 9, characterised in that it comprises the following steps:
- decoction or maceration in an alcohol, in particular methanol or ethanol, in order to remove polysaccharides, particularly starch,
- ultrafiltration of the hydroalcoholic solutions on an organic membrane of porosity such that its cut-off point would be 20000 Daltons for a globular protein ultrafiltered in aqueous solution,
- recuperation of the hydroalcoholic extract and evaporation to dryness, under reduced pressure, followed possibly by washing and recuperation of the dry residue,

12. A process according to Claim 10 or 11, characterised in that the recovered plant extract is concentrated then lyophilised or crystallised.

13. A process according to Claim 10 or Claim 11 characterised in that the initial plant material is composed of Cupressus sempervirens cones.

14. A composition according to any one of Claims 1 to 9, characterised in that it is non-cytotoxic.

15. A pharmaceutical composition, characterised in that it includes as an active ingredient, an effective quantity of at least one proanthocyanidol composition according to any one of Claims 1 to 9, in association with an acceptable pharmaceutical vehicle.

16. Use of a proanthocyanidol composition according to any one of Claims 1 to 9, for the manufacture of a medicine with anti-viral activity.

17. Use of a proanthocyanidol composition according to any one of Claims 1 to 3, for the manufacture of a medicine with anti-protease activity.

## Patentansprüche

1. Zusammensetzung auf Basis von Proanthocyanidolen, insbesonderen aus pflanzlicher Herkunft, gekennzeichnet dadurch, daß ein Gewichtsanteil von mindestens 50 % der Moleküle ein anscheinendes Molekulargewicht von größer als 2000 hat, wobei dieser Anteil zu mindest aus 40 % Proanthocyanidolen besteht und der Prozentsatz in Abhängigkeit von der Gerbung ausgedrückt wird, wobei diese Zusammensetzung im wesentlichen frei ist von:
- aromatischen Essenzen und insbesondere essentiellen Ölen, Harzen und anderen lipophilen Substanzen,
- polymerisierten Zuckern, wie beispielsweise Stärke, den Gummis, den Cellulosederivaten,
- oligomeren Zuckern, wie beispielsweise die Saccharose, die Glucose, die Levulose.

2. Zusammensetzung auf Basis von Proanthocyanidolen gemäß Anspruch 1, dadurch gekennzeichnet, daß sie im wesentlichen frei ist von:
- pflanzlichen Proteinen,
- Gallensäuren und Gallentanninen,
- Flavonoide, soweit sie nicht zur Familie der Flavan-Diole gehören,
- Flavan-Diole, Oligomere,
- Phenolsäuren.

3. Zusammensetzung nach Anspruch 1 oder 2, dadurch gekennzeichnet, daß sie mindestens 95 Trockengewichts-% Proanthocyanidole enthält.

4. Zusammensetzung nach einem der Ansprüche 1 bis 3, dadurch gekennzeichnet, daß die Proanthocyanidole ein Molekulargewicht zwischen etwa 2000 ± 500 und etwa 55000 ± 5000 haben.

5. Zusammensetzung nach einem der Ansprüche 1 bis 4, dadurch gekennzeichnet, daß sie Proanthocyanidole eines Molekulargewichts von etwa 45000 ± 5000 umfaßt.

6. Zusammensetzung nach einem der Ansprüche 1 bis 4, dadurch gekennzeichnet, daß sie Proanthocyanidole mit einem Molekulargewicht von etwa 5000 ± 500 umfaßt.

7. Zusammensetzung nach einem der Ansprüche 1 bis 4, dadurch gekennzeichnet, daß sie Proanthocyanidole mit einem Molekulargewicht von etwa 3000 ± 300 umfaßt.

8. Zusammensetzung nach einem der Ansprüche 1 bis 7, dadurch gekennzeichnet, daß diese Proanthocyanidole in polymerer Form entsprechend der folgenden Formel umfaßt: 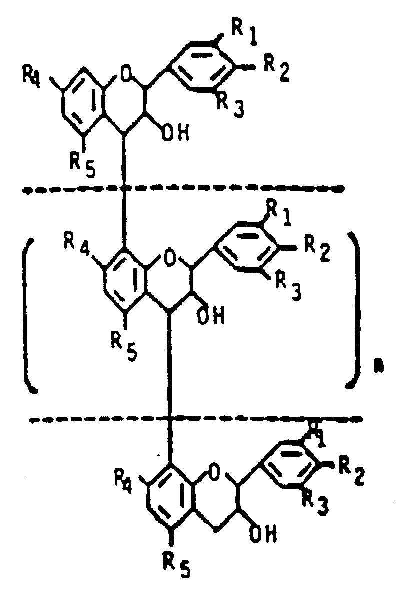 wobei n eine ganze Zahl oberhalb oder gleich 3 ist und vorzugsweise im Bereich zwischen 3 und 350 liegt,
R₁, R₂, R₃, R₄ und R₅ sind jeweils oder unabhängig voneinander H, OH oder OCH₃, wobei es sich bevorzugterweise um Polymere des Procyanidols handelt, in denen R₁, R₂, R₄ und R₅ Hydroxylgruppen und R₃ eine Wasserstoffgruppe sind, oder wobei es sich bevorzugterweise um ein Polymer des Prodelphinidols handelt, bei dem R₁, R₂, R₃, R₄ und R₅ Hydroxylgruppen sind, oder wobei es sich um ein Polymer des Promalvidols handelt, bei dem R₁ und R₃ Methoxygruppen und R₂, R₄ und R₅ Hydroxylgruppen sind.

9. Pflanzenextrakt nach einem der Ansprüche 1 bis 8, welches aus pflanzlichem Material gewonnen wurde, ausgewählt unter den Koniferen wie Cupressus sempervirens, Pinus maritima, den Akazien, den Mimosen.

10. Verfahren zur Herstellung einer Zusammensetzung auf Basis von Proanthocyanidolen aus einem Pflanzenteil, das Proanthocyanidole mit einem MG größer als 2000 nach einem der Ansprüche 1 bis 9 enthält, dadurch gekennzeichnet, daß es die folgenden Schritte umfaßt:
- Abkochen oder Auslaugen in einem Alkohol, insbesondere Methanol oder Ethanol, um Polysaccharide, insbesondere Stärke, zu eliminieren,
- Gewinnung der hydroalkoholischen Extrakte und Eindampfen zur Trockne unter vermindertem Druck, gegebenenfalls gefolgt von Waschen und Gewinnung des trockenen Rückstandes,
- Aufnehmen des trockenen Rückstandes und Filtration, um einen Teil der aromatischen Fraktion des Extrakts zu eliminieren,
- Extraktion in Gegenstromfahrweise mittels aufeinanderfolgend mindestens drei Lösungsmitteln zunehmender Polarität, insbesondere Ethylether, Ethylacetat, Butanol oder Methanol, insbesondere um verbleibende aromatische Essenzen zu eliminieren, den größten möglichen Teil der Oligomere mit einem MG unterhalb von 2000 und der Monomere des Proanthocyanidols und um in der alkoholischen Phase einen pflanzlichen Extrakt zu erhalten, der Proanthocyanidole mit einem Molekulargewicht oberhalb von 2000 enthält,
- gegebenenfalls Reinigung des erhaltenen Extraktes, um verbleibende Lösungsmittel zu eliminieren.

11. Verfahren zur Herstellung einer Zusammensetzung auf Basis von Proanthocyanidolen aus einem Pflanzenteil, enthaltend Proanthocyanidole mit einem MG oberhalb von 2000 nach einem der Ansprüche 1 bis 9 , dadurch gekennzeichnet, daß es die folgenden Schritte umfaßt:
- Abkochen oder Auslaugen in einem Alkohol, insbesondere Methanol oder Ethanol, um Polysaccharide, insbesondere Stärke, zu eliminieren,
- Ultrafiltration der hydroalkoholischen Lösungen durch eine organische Membran, die eine Porosität aufweist, welche eine Ausschlußgrenze oberhalb 20000 Dalton hat für ein globuläres Protein, das in einer wäßrigen Lösung ultrafiltriert wird,
- Gewinnung des hydroalkoholischen Extrakts und Eindampfen zur Trockne unter vermindertem Druck, gegebenenfalls gefolgt von einem Waschen und Gewinnen des trockenen Rückstandes.

12. Verfahren nach Anspruch 10 oder 11, dadurch gekennzeichnet, daß der pflanzliche gewonnene Extrakt lyophilisiert oder kristallisiert konzentriert wird.

13. Verfahren nach Anspruch 10 oder 11, dadurch gekennzeichnet, daß das pflanzliche Ausgangsmaterial aus Zapfen von Cupressus sempervirens besteht.

14. Zusammensetzung nach einem der Ansprüche 1 bis 9, dadurch gekennzeichnet, daß sie nicht cytotoxisch ist.

15. Pharmazeutische Zusammensetzung, dadurch gekennzeichnet, daß sie als Wirksubstanz eine wirksame Menge mindestens einer Zusammensetzung aus Proanthocyanidolen nach einem der Ansprüche 1 bis 9 zusammen mit einem pharmazeutisch annehmbaren Trägerstoff umfaßt.

16. Verwendung einer Zusammensetzung aus Proanthocyanidolen nach einem der Ansprüche 1 bis 9 zur Herstellung eines Arzneimittels mit antiviraler Aktivität.

17. Verwendung einer Zusammensetzung aus Proanthocyanidolen nach einem der Ansprüche 1 bis 3 zur Herstellung eines Medikaments mit Anti-Protease-Wirkung.
